# EUROPEAN PATENT APPLICATION

(11) **EP 1 623 996 A1**
(43) Date of publication of application: **08.02.2006**
(21) Application number: 04018722.1
(22) Date of filing: 06.08.2004
(51) Int. Cl.: C07K 16/30, G01N 33/53, C12N 15/73

(54) **Improved method of selecting a desired protein from a library**

(71) Applicant: Deutsches Krebsforschungszentrum Stiftung des öffentlichen Rechts, 69120 Heidelberg (DE)
(72) Inventor: Hoheisel, Jörg, 69168 Wiesloch (DE)
(74) Representative: Schüssler, Andrea

(57) **Abstract**

Described is an improved method of selecting a member of a specific binding pair (sbp) having a desired specify, preferably an antibody, from a library expressing said member of a sbp, preferably a phage-display antibody library.

## Description

The present invention relates to an improved method of selecting a member of a specific binding pair (sbp) having a desired binding specificity, preferably an antibody, from a library expressing said member of a sbp, preferably a phage antibody library.

Numerous attempts have already been made to obtain antibodies having a desired specificity in high yields and with good human compatibility, in particular as therapeutic agents. Antibody phage display libraries have become an important source for development of such antibodies. Large non-immune libraries serve as a single resource for the generation of antibodies to a wide range of self and non-self antigens, including tumor markers. Antibodies isolated from phage display libraries have typically been selected using purified antigens immobilized on plastic surfaces. Briefly, the phage antibody library is incubated in an antigen coated microtiter well and after washings, bound phages are eluted with low pH buffer. *E*. *coli* cells are infected with the eluted phages which are re-amplified in bacteria over night. The re-amplified phages are purified and incubated a second time with the antigen. Normally, after three or four panning rounds antigen specific antibodies are enriched from the phage library. Antigen specific antibodies have also been selected using cell lysates, fixed cells or living cells. These few successful selections have generally been done using libraries from immunized sources. In general, selection of antibodies by cell panning is limited by high background binding of nonspecific phages and relatively low binding of specific phages. In addition, most antibodies isolated this way bind to common features rather than epitopes, which are specific for the analysed sample. Also, strong binders frequently get lost during the process. However, the growing need for antibodies to be used in therapy/diagnosis, e.g., in early, preventive cancer diagnostics and tumor specific therapeutics puts a pressure on the current antibody selection techniques.

Thus, the technical problem underlying the present invention is to provide a method for selecting proteins, preferably antibodies, having desired specificities, which does not comprise the drawbacks of the former methods described above.

The solution to said technical problem is achieved by providing the embodiments characterized in the claims.

This solution is based on the enhancement of the selection procedure of cell- or tissue-type specific antibodies from libraries, preferably phage display libraries by the application of representational difference analysis (RDA; Lisitsyn et al., Science 259 (1993), 946-951). RDA is a combination of subtractive and kinetic enrichment in an iterative, PCR-coupled process, which has successfully been used to identify even small differences between complex DNA populations. Sequences present in one population - the tester - and less frequent or absent in the other - the driver - are selectively amplified by PCR. Taking advantage of the direct coupling of DNA sequence and antibody in phage display libraries, specific antibodies are identified this way. In turn, the isolated antibodies can then be used for the isolation of the cell- or tissue-type specific proteins.

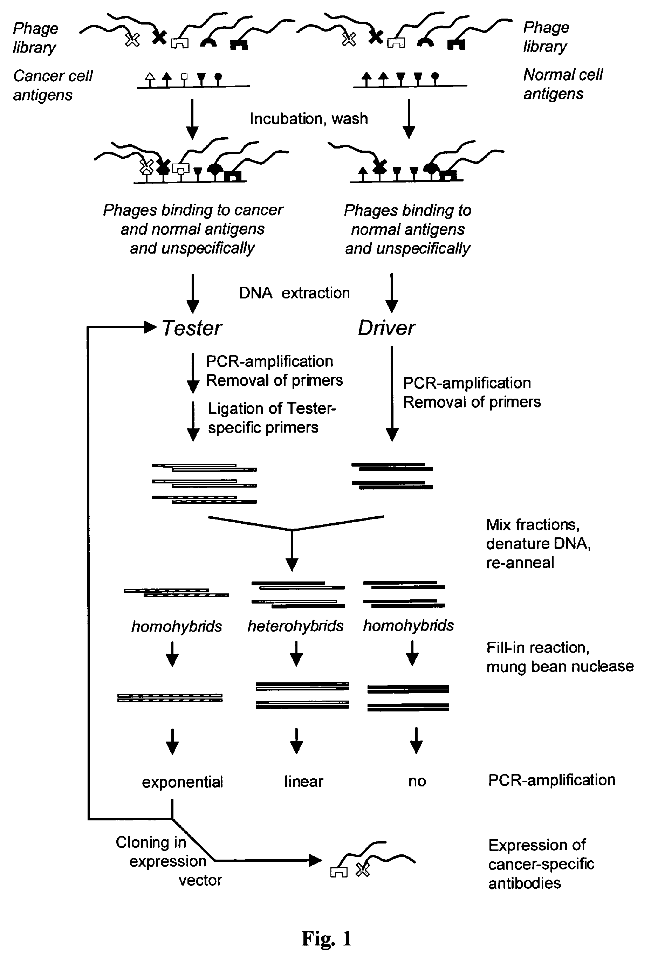

So far, the selection of cancer cell specific antibodies from phage display libraries requires laborious subtraction protocols to avoid the selection of irrelevant antibodies. For example, phage display libraries have to be incubated on normal cells before panning on tumor cells. In spite of such elaborate pre-incubations, irrelevant phages tend to cause high background levels. In the selection method of the present invention, RDA is utilized for the selection of specific antibodies from libraries, preferably phage display libraries, which bind, e.g., to tumor cells but not to normal cells or vice versa. A library, e.g., an antibody phage library, of high complexity is divided in two and incubated at the same time on slides made from tumor and normal tissue. No pre-incubation of the library is required. Non-binding phages are washed away by stringent washings. Subsequently, the DNA of the bound phages is isolated and the antibody encoding genes of the phages of either population are amplified by methods like PCR using primers with a 5'tag sequence that contains, e.g., a unique restriction site. After in vitro amplification, both amplicons are digested with this restriction endonuclease and new DNA-cassettes are ligated to the tester sample. Tester and driver DNA are mixed with driver DNA in excess, heat-denatured and re-annealed (Figure 1). Only self-re-annealed tester molecules have sequences at both termini that are complementary to the primers and are, thus, exponentially amplified. The resulting difference product can be enriched further by more RDA-cycles. Selectivity of the process can be varied by the ratios of driver:tester and kinetic parameters. The difference products are cloned into an expression vector for analyses of the antibody clones. By reversing the initial driver and tester population, also antibodies specific for the normal tissue can be selected.

Moreover, in a conventional panning procedure, where antigen-binding phages are eluted with low (or high) pH, there is a risk that very high affinity binders are not eluted and the best antibodies are lost. In addition, after elution, *E coli* cells are infected with eluted phages and grown for subsequent enrichment rounds. However, *E. coli* tolerates each antibody clone differently and good binders can also be lost during incubation because of a low expression level in bacteria. The RDA selection technique of the present invention circumvents both of these fundamental problems, since the elution and enrichment of the antibodies are performed at the DNA level. Since the antibodies are not eluted but only the DNA encoding them, very high affinity antibodies can be selected. Because PCR amplifies the different antibody genes similarly, the diverse expression levels of antibody clones in *E.coli* are not causing problems either. The biggest advantage compared to conventional panning, however, is the reduction of background and selection of only those antibodies which bind specifically to the target antigen. Although there might be a large number of antibodies binding to both tissue samples even unspecifically (which is a major problem in conventional panning experiments) the very high sensitivity and selectivity of RDA will select the differences between the two antibody populations. Moreover, the whole procedure is performed *in vitro* which makes it easy to automate.

### Brief description of the figures

Figure 1: Principle of the method of the present invention, shown exemplarily for a comparison of cancer and related normal tissue and use of a phage display library.

Thus, the present invention relates to a method of isolating a member of a specific binding pair (sbp) having a desired binding specificity, characterized by the following steps:
(a) incubating a library containing a diverse population of sbp members which are bound to the surface of a microorganism and comprise a binding domain for a complementary member of a sbp (i) with a mixture which contains a complementary sbp (tester) and (ii) with a mixture which does not contain a complementary member of an sbp (driver) or less amounts of the complementary member of the sbp compared to (i) under conditions suitable for binding of the complementary member of an sbp to said binding domain;
(b) washing away unbound microorganisms from step (a) (i) and (a) (ii) ;
(c) isolating the DNA or RNA from the bound microrganisms of step (b)(i) and (b)(ii);
(d) subjecting the DNA or RNA of step (c) to representational difference analysis (RDA); and, optionally,
(e) expressing the exponentially amplified DNA or RNA of step (d) to obtain the member of an sbp having the desired specificity.

If in the method of the present invention RNA is used for analysis, it has to be reversely transcribed according to well known methods prior to step (d).

Examples of mixtures of (i) and (ii) are tumor tissue and normal tissue.

The generation of a suitable library having sufficient diversity and method steps (a) to (c) can be carried out by the person skilled in the art by routine methods, e.g. by employing the techniques described in the publications regarding phage-display technology (see, e.g., EP-B1 0 589 877), ribosome display (Hanes and Plückthun, *Proc*. *Natl. Acad. Sci. USA* **94** (1997), 4937-4942), bacterial display (Georgiou et al., *Trends Biotechnol.* **11** (1993), 6-10) or yeast display (Kieke et al., *Protein Eng.* **10** (1997), 1303-1310).

Methods for carrying out steps (d) and (e) are, e.g., described in Lisitsyn et al., Science **259** (1993), 946-951.

For the generation of the libraries, cells are transfected with the DNAs encoding the diverse population of sbp members by means of standard methods known to the person skilled in the art (Sambrook and Russell: Molecular Cloning, a laboratory manual, 3^{rd} edition, 2001, ISBN 0-87969-577-3; Chapter 16) such as electroporation (AGS company / Hybaid or BioRad, Handbuch der Elektroporatoren [manual of electroporators] BTX or BioRad GenePulser) or the transfection, e.g. with LipfectAMINE™ 2000 Reagent (Invitrogen #1668-027), with DMRIE-C Reagent (Invitrogen #10459-014), or LipofectAMINE™ Reagent (Invitrogen #18324-012), FuGENE 6 Transfection Reagent (Roche #1815091), DOTAP Liposomal Transfection Reagent (Roche #1811177), or DOSPER Liposomal Transfection Reagent (Roche #1811169).

The person skilled in the art also knows methods as to the contacting of the member of a sbp with the complementary member of an sbp, e.g., the antigen with an antibody library (see, e.g., Liddell and Weeks, 1996, "Monoclonal Antibodies: Principles and Practice." Spektrum-Verlag ISBN 3827400481; Goding, J. W., 1996, "Production and Application of Monoclonal Antibodies in Cell Biology, Biochemistry and Immunology. Third Edition. Published by Academic Press Limited, 24-28 Oval Road, London NW1 7DX; ISBN 0-12-287023-9). These methods have also been described in detail for surface-presented recombinant antibodies from phage libraries *(inter* alia de Kruif et al., 1995, PNAS 92, 3938-3942) and from bacterial libraries (Fuchs et al., 1996, J. of Immunotechnology. 2, 97-102).

Several kinds of microorganisms are suitable for the method of the present invention such as bacteria, yeasts, phages etc. (see, e.g., as regards the phage display, EP-B1 0 589 877), ribosome display (Hanes and Plückthun, *Proc. Natl. Acad. Sci. USA* **94** (1997), 4937-4942), bacterial display (Georgiou et al., *Trends Biotechnol.* **11** (1993), 6-10) or yeast display (Kieke et al., *Protein Eng*. **10** (1997), 1303-1310).

The term "microorganism" as used herein also comprises parts of a microorganism, e.g., ribosomes. In a preferred embodiment of the method of the present invention, said microorganism is a phage. In case of a phage-display antibody library the genome of the phage particles is manipulated by inserting the DNAs encoding the members of an sbp into phage genes encoding a phage coat protein. Thus, the member of the sbp is presented as a fusion protein on the phage surface. The most abundantly used display system involves fusing the proteins to be presented to the minor protein pIII that is located on the tip of the phage surface.

As used herein, the term "diverse population of sbp members" refers not only to diversity that can exist in the natural population of cells or microorganisms, but also to diversity that can be created by artificial mutation in *vitro* or *in vivo.* Mutation *in vitro* may, e.g., involve random mutagenesis using oligonucleotides having random mutations of the sequence desired to be varied. *In vivo* mutagenesis may, e.g., use mutator strains of host microorganisms to harbor the DNA or splice variations.

As used herein, the term "specific binding pair" (sbp) means a pair of molecules (each being a member of a specific binding pair) which are naturally derived or synthetically produced. One of the pair of molecules has an area on its surface, or a cavity which specifically binds to, and is therefore defined as complementary with a particular spatial and polar organization of the other molecule, so that the pair have the property of binding specifically to each other. Examples of types of specific binding pairs include antigen-antibody, biotin-avidin, hormone-hormone receptor, receptor-ligand, enzyme-substrate, IgG-protein A.

In a further preferred embodiment of the method of the present invention, said member of the sbp is an antibody and said complementary sbp is an antigen. The term "antibody" as used herein describes an immunoglobulin whether natural or partly or wholly synthetically produced. This term also covers any protein having a binding domain which is homologous to an immunoglobulin binding domain. These proteins can be derived from natural sources, or partly or wholly synthetically produced. Examples of antibodies are the immunoglobulin isotypes and the Fab, F(ab¹)_{2'} scFv, Fv, dAb and Fd fragments.

Preferably, the representational difference analyis (RDA; step (d)) is characterized by the following steps:
(d₁) attaching (e.g. by ligation) specific oligonucleotides (adaptors) to both ends of the tester DNA;
(d₂) hybridizing tester DNA and driver DNA together;
(d₃) filling in the truncated ends of the hybridized DNAs, e.g., by use of a DNA polymerase; optionally single-stranded, unhybridized DNA regions are digested, preferably by use of Mung Bean Nuclease; and
(d₄) subjecting the DNAs of step (d₃) to exponential amplification using the same oligonucleotides as has been used for step (d₁) as primers. Only self-re-annealed tester DNAs have complementary oligonucleotide sequences at both ends and can, thus, subsequently be amplified at an exponential rate.

The preferable ratio of tester DNA to driver DNA in step (d₂) is at any ratio and can be determined by the person skilled in the art by routine experimentation, but is preferably in the range of 1:10 to 1:10⁴.

It is preferred to carry out several rounds of RDA in order to enhance the selectivity. Thus, in a more preferred embodiment of the method of the present invention, step (d) is repeated at least once. When repeating step (d) new oligonucleotides (adaptors) are attached to the enriched tester DNA from the previous round and, preferably, selection pressure is increased by decreasing the amounts of tester DNA in the hybridization step, preferably down to ratios of 1:10⁶.

Any method for exponential *in vitro* amplification can be used in the method of the present invention, e.g., PCR, RT/PCR, LCR, NASBA, with PCR being preferred, however.

The specific members of an sbp, e.g., antibodies, obtained by the method of the invention are useful for many diagnostic/therapeutic purposes, e.g., for the identification of differences between two complex protein populations.

The invention is explained by the below examples.

### Example 1: Selection of a specific antibody from a phage display library by use of RDA

Initially, RDA was adapted and optimized to the selection of antibodies from phage libraries. The VTT library was pre-incubated with tumor antigens for the isolation of antigen-binding phages. The original library was divided in two and one half was spiked with different amounts of the antigen binding phages to create a tester. RDA was then performed on the two phage populations.

In a second experiment, microtiter-wells were coated with the tumor antigen and blocked with bovine serum albumin (BSA) (tester). The control-well was only BSA-blocked (driver). The antibody phage display library was spiked with various amounts of tumor antigen binding phages and incubated in the antigen-coated and control-wells. Non-binding phages were washed away by standard procedures and DNA of the bound phages was extracted from the wells. By application of RDA, DNA encoding the antigen-binding antibody was enriched, following the basic protocol of Frohme et al., *Genome Res*. **11** (2001), 901-903.

In a third type of experiment, antigen specific antibodies were selected using RDA from a phage display library which was known from the previous experiments to contain tumor antigen-binding antibodies. Since by use of the method of the present invention the background of antibodies that bind to both the tumor and normal cells is removed efficiently by the RDA procedure, highly tumor-specific antibodies were obtained.

## Claims

1. A method of isolating a member of a specific binding pair (sbp) having a desired binding specificity, **characterized by** the following steps:
(a) incubating a library containing a diverse population of sbp members which are bound to the surface of a microorganism and comprise a binding domain for a complementary member of a sbp (i) with a mixture which contains a complementary sbp (tester) and (ii) with a mixture which does not contain a complementary member of an sbp (driver) or less amounts of the complementary member of the sbp compared to (i) under conditions suitable for binding of the complementary member of an sbp to said binding domain;
(b) washing away unbound microorganisms from step (a) (i) and (a) (ii) ;
(c) isolating the DNA or RNA from the bound microrganisms of step (b)(i) and (b)(ii);
(d) subjecting the DNA or RNA of step (c) to representational difference analysis (RDA); and, optionally,
(e) expressing the exponentially amplified DNA or RNA of step (d) to obtain a member of an sbp having the desired specificity.

2. The method of claim 1, wherein said microorganism is a phage.

3. The method of claim 1 or 2, wherein said member of the sbp is an antibody and said complementary sbp is an antigen.

4. The method of any one of claims 1 to 3, wherein step (d) is **characterized by** the following steps:
(d₁) attaching specific oligonucleotides (adaptors) to both ends of the tester DNA;
(d₂) hybridizing tester DNA and driver DNA together;
(d₃) filling in the truncated ends of the hybridized DNAs and, optionally, digesting single-stranded, unhybridized DNA regions; and
(d₄) subjecting the DNAs of step (d₃) to exponential amplification using the same oligonucleotides as has been used for step (d₁) as primers.

5. The method of claim 4, wherein in step (d₂) the tester DNA to driver DNA is at any ratio.

6. The method of any one of claims 1 to 5, wherein step (d) is repeated at least once.

7. The method of any one of claims 1 to 6, wherein exponential amplification is carried out by PCR.

8. Use of a member of an sbp obtainable by the method of any one of claims 1 to 7 for identifying differences between at least two complex protein populations.

9. The use according to claim 8, wherein said member of an sbp is an antibody.

10. The method according to any one of claims 3 to 7 or the use of claim 9, wherein said antibody is a tumor specific antibody
